# EUROPÄISCHE PATENTSCHRIFT

(11) **EP 0 668 853 B1**
(45) Veröffentlichungstag und Bekanntmachung des Hinweises auf die Patenterteilung: **02.04.1997**
(21) Anmeldenummer: 93924562.7
(22) Anmeldetag: 02.11.1993
(51) Int. Cl.: C07C 69/593, C07C 69/34, A61K 7/40

(54) **HAUTKONDITIONIERENDE BERNSTEINSÄURE-DERIVATE**
SUCCINIC ACID DERIVATIVES FOR USE AS SKIN CONDITIONERS
DERIVES DE L'ACIDE SUCCINIQUE UTILISES COMME AGENTS POUR LE CONDITIONNEMENT DE LA PEAU

(30) Priorität: 11.11.1992 DE 4238032
(43) Veröffentlichungstag der Anmeldung: 30.08.1995
(73) Patentinhaber: Henkel Kommanditgesellschaft auf Aktien, 40191 Düsseldorf (DE)
(72) Erfinder: EIERDANZ, Horst, D-40723 Hilden (DE); BUSCH, Peter, D-40699 Erkrath (DE); TESMANN, Holger, D-41363 Jüchen (DE); KNÖRR, Walter, D-40723 Hilden (DE); WACHTER, Rolf, D-40595 Düsseldorf (DE)
(86) Internationale Anmeldenummer: EP9303044
(87) Internationale Veröffentlichungsnummer: WO9411333

(56) Entgegenhaltungen:
- EP-A- 0 107 199
- US-A- 3 968 310

## Beschreibung

Die Erfindung betrifft neue Bernsteinsäure-Derivate mit lipidanaloger Struktur, die sich als hautfeuchtigkeitsregulierende Komponente in kosmetischen und dermatologischen Zubereitungen eignen.

Zur Gesunderhaltung der Haut und zur Förderung eines kosmetisch ansprechenden, glatten Aussehens und geschmeidigen Hautgefühls gehört in erster Linie die Aufrechterhaltung des natürlichen Feuchtigkeitsgehalts des stratum corneum. Es sind viele hautbefeuchtende und feuchthaltende Zusätze für kosmetische und therapeutische Hautbehandlungsmittel bekannt geworden, bei welchen es sich meist um wasserlösliche oder wasserbindende Stoffe, oft sogar um hygroskopische Verbindungen handelt. Diese Stoffe haben meist den Nachteil, daß sie bei häufigem Gebrauch zu einer noch stärkeren Austrocknung der Haut beitragen.

Paraffinöle, Triglyceridöle und Wachse zeichnen sich durch eine zu stark okklusive Wirkung aus, die den transepidermalen Wasseraustausch zu sehr hemmt.

in jüngster Zeit ist die hautfeuchtigkeitsregulierende Wirkung der Hornschichtlipide der menschlichen Haut und auch von synthetischen Analoga dieser Lipidstoffe bekannt geworden (J. Soc. Cosmet. Chem. 40 (1989), 273 - 285). Es handelt sich dabei z.B. um N-Acyl-Sphingosine und um Fettsäureamide von 1-N-Hydroxyethylamino-2-hydroxy-3-alkoxypropan (vgl. z.B. EP 0 277 641, EP 0 455 429).

Es wurde nun gefunden, daß Alkyl- und Alkenylbernsteinsäure-monoester-alkoxylate, die synthetisch leicht zugänglich sind, ebenfalls eine den natürlichen und synthetischen Ceramiden analoge Wirkung haben.

Gegenstand der Erfindung sind Bernsteinsäure-Derivate der Formel I

R¹O(CₙH₂ₙO)ₓCO - CHR² - CHR³ - CO(OCₙH₂ₙ)_{y}OR⁴ (I),

in der R¹ eine Alkyl-, Alkenyl-, Mono- oder Dihydroxyalkyl- oder eine Hydroxyalkenylgruppe mit 6 - 22 C-Atomen, eine der Gruppen R² und R³ Wasserstoff und die andere eine Alkyl- oder Alkenylgruppe mit 12 - 22 C-Atomen, n = 2 oder 3, x und y mittlere Oxalkylierungsgrade, und zwar x von 0 - 20 und y von 1 - 20 sind und R⁴ Wasserstoff oder eine Gruppe R¹O - (CₙH₂ₙO)ₓ - CO - CHR² - CHR³ - CO - darstellt oder die gleiche Bedeutung wie R¹ hat.

Die erfindungsgemäßen Bernsteinsäure-Derivate eignen sich vorzüglich zur Verwendung als hautfeuchtigkeitsregulierende Komponente zur Herstellung von kosmetischen oder therapeutischen Zubereitungen zur Behandlung der Haut. Sie erhöhen aufgrund ihres Lipidcharakters die Glätte und Geschmeidigkeit der Haut und regulieren den transepidermalen Wasserverlust ohne jedoch eine wasserundurchlässige Barriere zu bilden. Sie beugen der durch klimatische und tensidische Einwirkung bedingten Hautaustrocknung vor und tragen dazu bei, daß die Haut ein glattes, jugendliches Aussehen behält.

Die Herstellung der erfindungsgemäßen Bernsteinsäure-Derivate erfolgt nach an sich bekannten Verfahren der präparativen Chemie ausgehend von den bekannten Alkenylbernsteinsäure-anhydriden, die durch En-Addition von Maleinsäureanhydrid an Olefine zugänglich sind. Ein solches Bernsteinsäureanhydrid der allgemeinen Formel II 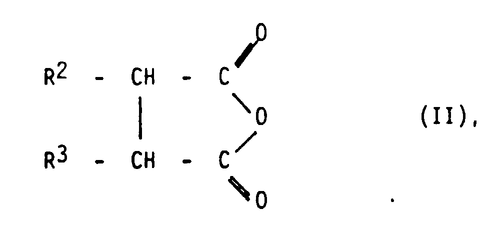 in der eine der Gruppen R² und R³ Wasserstoff und die andere eine Alkenylgruppe mit 12 - 22 C-Atomen ist, wird mit einem Alkohol der Formel R¹ - OH zum Monoester umgesetzt, der Monoester wird dann mit (x + y) mol Ethylenoxid (n = 2) oder Propylenoxid (n = 3) oxalkyliert. R¹, R², R³, n, x und y haben dabei die für die Formel I angegebene Bedeutung. Wenn man wünscht, daß die Gruppe R² bzw. R³ eine Alkylgruppe ist, so kann man das Bernsteinsäureanhydrid der Formel II, den Monoester oder das Oxalkylat zur Absättigung der enthaltenen Doppelbindungen hydrieren.

Als Alkohole der Formel R¹OH eignen sich z.B. Fettalkohole mit 6 - 22 C-Atomen, bevorzugt z.B. Stearyl, Arachidyl-, Behenyl-, Oleyl-, Linoleyl- und Erucaalkohol. Geeignet sind auch Alkandiole und Alkantriole mit 6 - 22 C-Atomen wie z.B. Ricinolylalkohol, 9-Hydroxystearylalkohol und Alkantriole wie z.B. 9,10-Dihydroxystearylalkohol.

Die Oxalkylierung des Monoesters wird bevorzugt in Gegenwart basischer Katalysatoren wie z.B. LiOH, NaOH, KOH, NaOCH₃, KOCH₃ und anderen für die Umsetzung von Ethylenoxid oder Propylenoxid mit reaktiven Hydroxylgruppen bekannten Katalysatoren durchgeführt. Bevorzugt arbeitet man bei Temperaturen von 100 - 200°C und einem leichten Überdruck von 1 - 10 bar und mit einem Überschuß an Ethylenoder Propylenoxid. Der Monoester der Formel I addiert ein erstes mol Alkylenoxid auch in Abwesenheit eines Katalysators an die freie Carboxylgruppe (y = 1); wenn man mehr als ein mol Alkylenoxid anlagern will, so muß man unter Zusatz eines Alkoxylierungskatalysators eine zweite Alkoxylierungsstufe anschließen oder von vornherein die Alkoxylierung in Gegenwart eines Katalysators durchführen.

Bei der Oxalkylierung in Gegenwart von Katalysator wird Alkylenoxid auch in die Esterbindung des Monoesters eingeschoben; auf diese Weise kommt es zur Anlagerung der in Formel I mit x gekennzeichneten Alkoxygruppen. Schließlich kommt es bei der Oxalkylierung auch zu einer teilweisen Umesterung des Alkoxylats der Formel I mit R⁴ = H unter Bildung komplexer Ester, in welchen R⁴ = R¹O(CₙH₂ₙO)ₓ-CO-CHR²-CHR³-CO- ist oder die gleiche Bedeutung wie R₁ hat. Monoester-Ethoxylate der Formel I, in der R⁴ = H ist, stellen jedoch die Hauptkomponente des Reaktionsgemischs dar.

Alternativ kann man den Monoester auch in das Alkali- oder Erdalkalisalz überführen und dieses dann in einem inerten Lösungsmittel oxalkylieren.

Von den erfindungsgemäßen Bernsteinsäure-Derivaten der Formel I eignen sich bevorzugt solche, in denen R¹ eine Alkylgruppe mit 16 - 22, insbesondere von 20 - 22 C-Atomen ist, als hautfeuchtigkeitsregulierende Lipidkomponente zur Herstellung von kosmetischen und therapeutischen Zubereitungen zur Behandlung der Haut. Ganz besonders bevorzugt sind solche Bernsteinsäure-Derivate der Formel I, worin n = 2 und (x + y) = 1 - 20 ist.

Die neuen Verbindungen sind öllöslich und weisen eine gewisse Grenzflächenaktivität aufgrund der polaren Gruppen im Molekül auf. Sie sind daher besonders leicht in kosmetische und dermatologische Formulierungen einzuarbeiten. Bevorzugt werden die erfindungsgemäßen Bernsteinsäure-Derivate in einer Menge von 1 - 10 Gew.-% in einen geeigneten kosmetischen oder dermatologischen Träger eingearbeitet.

Als Träger eignen sich dabei sowohl kosmetische Öle und Aerosolzubereitungen als auch wäßrige, emulsionsförmige Systeme. In üblichen Ölkomponenten, z.B. in Paraffinöl, Pflanzenölen und Fettsäureestern lösen sie sich klar auf. Sie lassen sich sowohl allein als auch im Gemisch mit üblichen kosmetischen Ölkomponenten unter Verwendung bekannter Emulgatoren mit Wasser emulgieren, wobei je nach dem gewählten Emulgator und Emulgierverfahren Öl-in-Wasser-, Wasser-in-Öl-oder gemischte Emulsionssysteme erhalten werden können. Die erfindungsgemäßen Bernsteinsäure-Derivate können auf diese Weise problemlos z.B. in Hautcremes, Lotionen, Hautöle, Sonnenschutzmittel, Körper-Aerosole, Haarwässer und Badeöle eingearbeitet werden.

Die folgenden Beispiele sollen den Erfindungsgegenstand näher erläutern.

### Beispiele

1. Herstellungsbeispiele
1.1 Hexadecenylbernsteinsäure-monoerucylester-Oxethylat (4,5 EO) (R¹ = Erucyl-, R² = H, R³ = Hexadecenyl-, n = 2, (x + y) = 4,5)
   551 g (1,7 mol) Hexadecenylbernsteinsäureanhydrid (kurzwegdestilliert) wurden mit 649,2 g (2 mol) Erucaalkohol 0,5 h bei 60°C gerührt.
   Kennzahlen: SZ 83 OHZ 18 VZ 153 JZ 78
   2-Stufen-Ethoxylierung des Monoesters
   * 500 g (0,77 mol) des Monoesters wurden mit 211,6 g (4,8 mol) Ethylenoxid 8 h bei 180°C/5 bar (N₂) gerührt. Ausbeute: 535,9 g gelbe, viskose Flüssigkeit Es wurde ca. 1 mol EO pro mol Halbester aufgenommen.
      Kennzahlen: SZ 3,2
   * 232,3 g vorethoxylierter Halbester (0,33 mol) wurden mit 1,2 g (30 % in Methanol) Natriummethylat versetzt und mit 84,1 g (1,9 mol) Ethylenoxid 2 h bei 150°C/5 bar (N₂) nachethoxyliert. Das dunkelgefärbte Rohprodukt wird über Tonsil / A-Kohle filtriert.
      Ausbeute: 291,4 hellgelbe, viskose Flüssigkeit.
      Es wurden ca. 3,6 mol EO pro mol Halbesterethoxylat zusätzlich aufgenommen.
      Der Gesamtethoxylierungsgrad liegt bei 4,5 mol EO pro mol Halbester.
      Kennzahlen: SZ 0,4 OHZ 44 JZ 53 VZ 100
1.2 Hexadecylbernsteinsäure-monostearylester-Oxethylat (7,6 EO) (R¹ = Stearyl-, R² = H, R³ = Hexadecyl-, n = 2, (x + y) = 7,6)
   275,7g (0,85 mol) Hexadecenylbernsteinsäureanhydrid (kurzwegdestilliert) wurden mit 297,6 g (1,1 mol) Stearylalkohol 1 h bei 80°C gerührt. Es resultiert ein hellgelber Feststoff.
   Kennzahlen: SZ 79 OHZ 16,5 VZ 166 JZ 36
   Hydrierung:
   529,4 g (0,9 mol) Monostearylester wurden an 26,5 g Palladium auf A-Kohle (5 % Pd) 3 h bei 80°C/10 bar H₂ (bis zum Ende der Wasserstoffaufnahme) hydriert. Filtration über Hyflo liefert 409,6 g hellgelben Feststoff.
   Kennzahlen: JZ 0,3
   Ethoxylierung des hydrierten Esters:
   379,4 g (0,64 mol) des hydrierten Monoesters wurden mit 1,9 g (12 mol-%) LiOH und 250,4 g (5,7 mol) Ethylenoxid 8 h bei 180°C / 5 bar (N₂) gerührt. Das Rohprodukt wurde mit 2 % (bezogen auf Gesamtansatz) Tonsil 1 h bei 90°C gerührt und anschließend über Hyflo filtriert.
   Ausbeute: 617,5 g gelber Feststoff
   Es wurden ca. 7,6 mol EO pro mol Halbester aufgenommen.
   Kennzahlen: SZ 0,8 OHZ 78 VZ 94
1.3 Hexadecylbernsteinsäure-monobehenylester-Oxethylat (7,3 EO) R¹ = Behenyl-, R² = H, R³ = Hexadecyl-, n = 2, (x + y) = 7,3)
   275,7 g (0,85 mol) Hexadecenylbernsteinsäureanhydrid (kurzwegdestilliert) wurden mit 359,3 g (1,1 mol) Behenylalkohol (Stenol 1822 A) 1 h bei 80°C gerührt.
   Kennzahlen: SZ 74 OHZ 11,7 VZ 152 JZ 30
   Hydrierung:
   529,4 g (0,8 mol) Monobehenylester wurden an 26,5 g Palladium auf A-Kohle (5 % Pd) 3 h bei 80°C / 10 bar H₂ (bis zum Ende der Wasserstoffaufnahme) hydriert. Filtration über Hyflo liefert 431,8 g hellgelben Feststoff.
   Kennzahlen: JZ 1,0
   Ethoxylierung des hydrierten Esters:
   394,8 g (0,6 mol) des hydrierten Monoesters wurden mit 2 g (12 mol-%) LiOH und 229,3 g (5,2 mol) Ethylenoxid 10 h bei 180°C / 5 bar (N₂) gerührt. Das Rohprodukt wurde mit 2 % (bezogen auf Gesamtansatz) Tonsil 1 h bei 90°C gerührt und anschließend über Hyflo filtriert.
   Ausbeute: 604,4 g gelber Feststoff
   Es wurden ca. 7,3 mol EO pro mol Halbester aufgenommen.
   Kennzahlen: SZ 0,4 OHZ 73 VZ 92
1.4 Hexadecylbernsteinsäure-monostearylester-Oxethylat (1,3 EO) (R¹ = Stearyl-, R² = H, R³ = Hexadecyl-, n = 2, (x + y) = 1,3)
   275,5 g (0,85 mol) Hexadecenylbernsteinsäureanhydrid (kurzwegdestilliert) wurden mit 297,6 g (1,1 mol) Stearylalkohol 1 h bei 80°C gerührt. Es resultiert ein hellgelber Feststoff.
   Kennzahlen: SZ 84 OHZ 20 VZ 167 JZ 37
   Ethoxylierung des Halbesters:
   382,2 g (0,65 mol) des Monoesters wurden mit 2,1 g (12 mol-%) LiOH und 46,2 g (1,05 mol) Ethylenoxid 5 h bei 180°C / 5 bar (N₂) gerührt. Das Rohprodukt wurde mit 2 % (bezogen auf Gesamtansatz) Tonsil 1 h bei 90°C gerührt und anschließend über Hyflo filtriert.
   Ausbeute: 423,6 gelber Feststoff
   Es wurden ca. 1,3 mol EO pro mol Halbester aufgenommen.
   Kennzahlen: SZ 0,5 OHZ 89 VZ 143 JZ 33
   Hydrierung:
   200 g (0,3 mol) Monostearylesterethoxylat wurden an 1 g Palladium auf A-Kohle (5 % Pd) 3 h bei 80°C / 10 bar H₂ (bis zum Ende der Wasserstoffaufnahme) hydriert. Filtration über Hyflo liefert 161 g hellgelben Feststoff.
   Kennzahlen: JZ 3,3
   Das Produkt ist deutlich heller als gemäß Beispiel 1.3.
1.5 Hexadecylbernsteinsäure-monobehenylester-Oxethylat (7,5 EO) R¹ = Behenyl-, R² = H, R³ = Hexadecyl-, n = 2, (x + y) = 7,5
   Hydrierung des Hexadecenylbernsteinsäureanhydrids
   300 g (0,93 mol) Hexadecenylbernsteinsäureanhydrid (destilliert) wurden mit 6 g Ni-Katalysator (22 % Ni auf Träger in Fettmatrix) 4 h bei 180°C/10 bar H₂ (bis zum Ende der Wasserstoffaufnahme) hydriert. Filtration über Hyflo liefert 280 g hellgelben Feststoff.
   Kennzahlen: JZ 2,9
   Alkoholyse des hydrierten Anhydrids mit anschließender Ethoxylierung des Halbesters
   261 g (0,8 mol) Hexadecylbernsteinsäureanhydrid wurden mit 271 g (0,84 mol) Behenylalkohol (Stenol 1822A) 30 min bei 80°C unter N₂ gerührt. Das Reaktionsprodukt wurde mit 2,7 g (0,5 Gew.-%) pulverisierter KOH und anschließend bei 140°C/4,5 bar (N₂) portionsweise (während ca. 80 min) mit 284 g (6,45 mol) Ethylenoxid versetzt und 1 h bei 140°C nachreagiert. Das Rohprodukt wurde unter N₂ mit 2 % (bezogen auf Gesamtansatz) Tonsil / Aktivkohle (3 : 1 w/w) 1 h bei 90°C gerührt und bei max. 60°C über Hyflo filtriert.
   Ausbeute: 777 g gelber Feststoff
   Es wurden ca. 7,5 mol EO pro mol Halbester aufgenommen.
   Kennzahlen: SZ 0,3 OHZ 64 VZ 111

2. Anwendungsbeispiele
2.1 O/W-Handpflegecreme 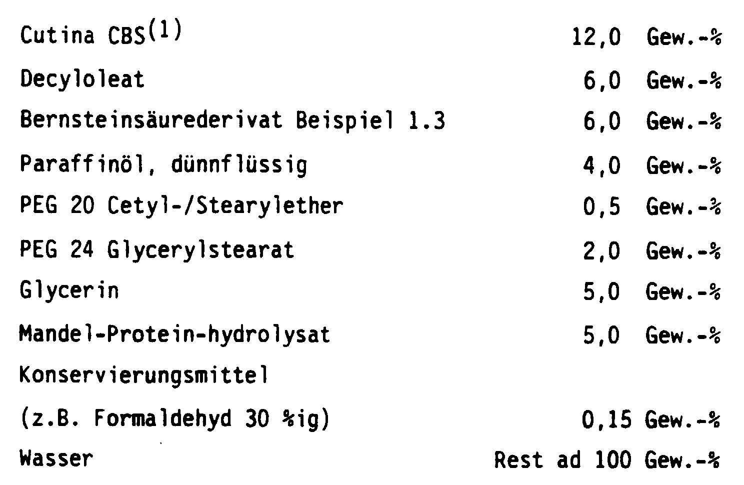
2.2 O/W-Softcreme

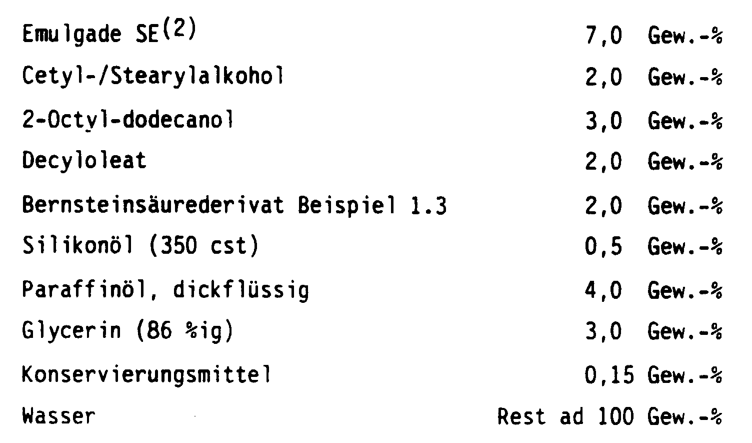

2.3 O/W-Nähr- und Pflegecreme 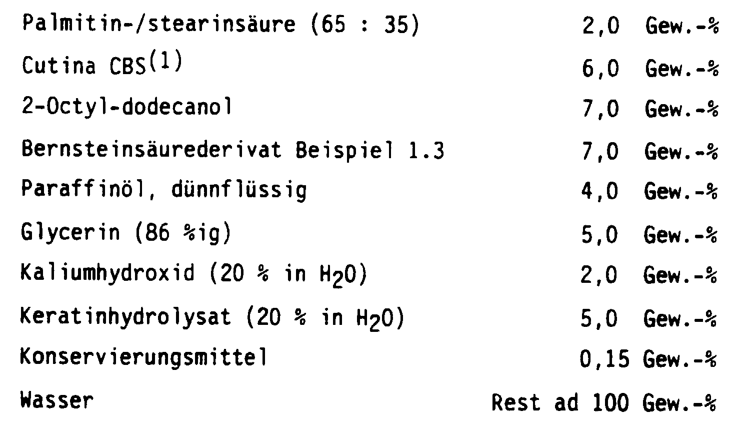
2.4 OW-Hautcreme

| | |
|---|---|
| Glycerinmono-/dipalmitat/stearat | 6,0 Gew.-% |
| PEG 12-cetyl-/stearylether | 1,0 Gew.-% |
| PEG 20-cetyl-/stearylether | 1,0 Gew.-% |
| Cetyl-/stearylalkohol | 2,0 Gew.-% |
| Bernsteinsäurederivat Beispiel 1.3 | 6,0 Gew.-% |
| Decyloleat | 3,0 Gew.-% |
| Cetyl-/stearyl-isononanoat | 3,0 Gew.-% |
| Paraffinöl, dickflüssig | 4,0 Gew.-% |
| Glycerin 86 %ig | 5,0 Gew.-% |
| Konservierungsmittel | 0,15 Gew.-% |
| Wasser | Rest ad 100 Gew.-% |

2.5 O/W-Hautcreme 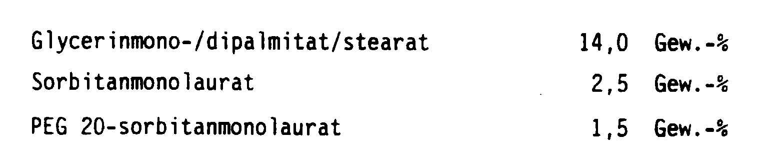 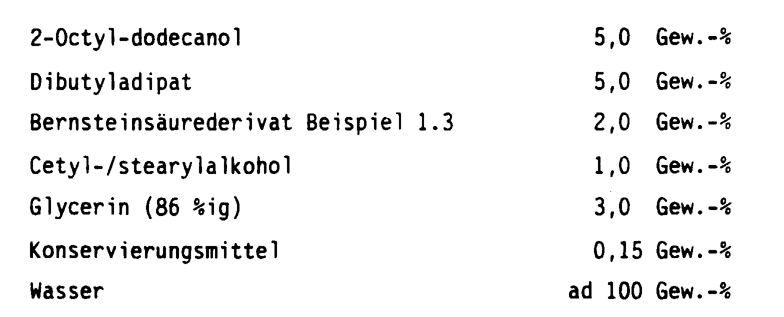
2.6 O/W-Sonnenschutzcreme 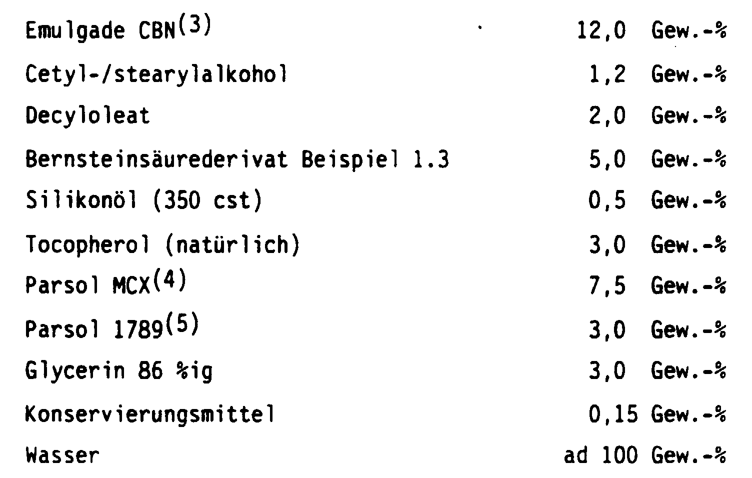
2.7 W/O-Pflegecreme 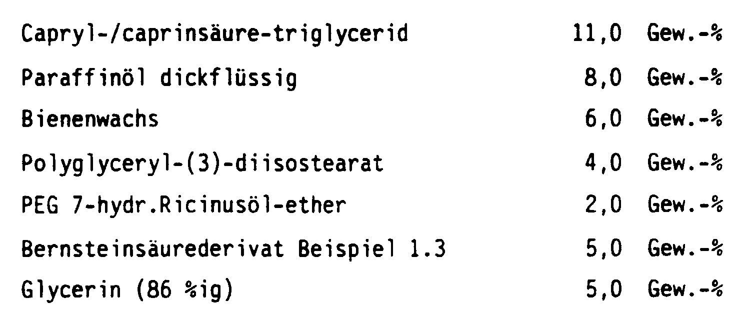 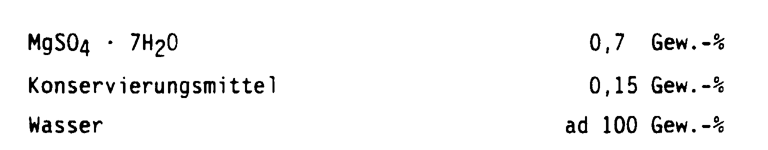
2.8 W/O-Nachtcreme

| | |
|---|---|
| Polyglyceryl-(3)-diisostearat | 4,0 Gew.-% |
| PEG 7-hydr. Ricinusöl-ether | 3,0 Gew.-% |
| Cetyl-/stearylalkohol | 1,0 Gew.-% |
| Bienenwachs | 5,0 Gew.-% |
| Zinkstearat | 0,5 Gew.-% |
| Paraffinöl, dünnflüssig | 7,0 Gew.-% |
| 1,3-Bis-(2-ethylhexyl)-cyclohexan | 6,0 Gew.-% |
| Bernsteinsäurederivat Beispiel 1.3 | 10,0 Gew.-% |
| Glycerin (86 %ig) | 5,0 Gew.-% |
| MgSO₄ · 7H₂O | 0,7 Gew.-% |
| Mandel-Protein-hydrolysat (22 %ig in H₂O) | 2,5 Gew.-% |
| Konservierungsmittel | 0,15 Gew.-% |
| Wasser | ad 100 Gew.-% |

2.9 W/O-Pflegelotion

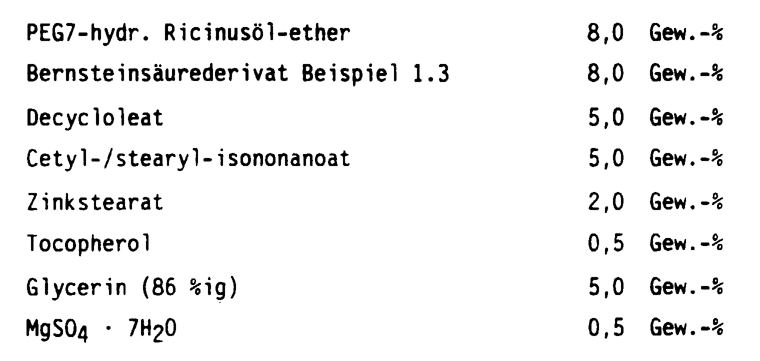

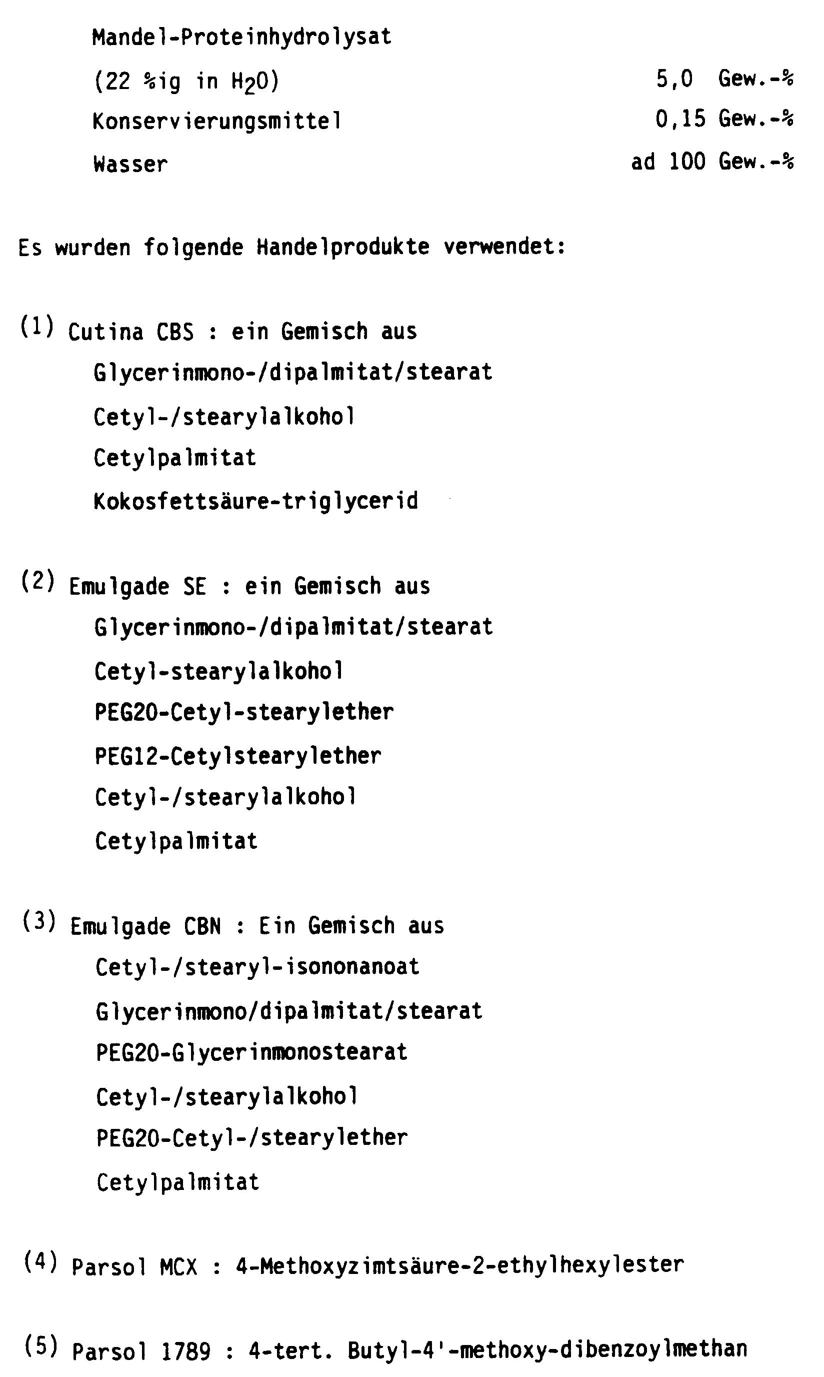

## Patentansprüche

1. Bernsteinsäure-Derivate der Formel I
R¹O(CₙH₂ₙO)ₓCO - CHR² - CHR³ - CO(OCₙH₂ₙ)_{y}OR⁴ (I),
in der R¹ eine Alkyl-, Alkenyl-, Mono- oder Dihydroxyalkyl- oder eine Hydroxyalkenylgruppe mit 6 - 22 C-Atomen, eine der Gruppen R² und R³ Wasserstoff und die andere eine Alkyl- oder Alkenylgruppe mit 12 - 22 C-Atomen, n = 2 oder 3, x und y mittlere Oxalkylierungsgrade, und zwar x von 0 - 20 und y von 1 - 20 und R⁴ Wasserstoff oder eine Gruppe R¹O - (CₙH₂ₙO)ₓ - CO - CHR² - CHR³ - CO - darstellt oder die gleiche Bedeutung wie R¹ hat.

2. Bernsteinsäure-Derivate gemäß Anspruch 1, dadurch gekennzeichnet, daß R¹ eine Alkylgruppe mit 16 - 22 C-Atomen ist.

3. Bernsteinsäure-Derivate gemäß Anspruch 1 oder 2, dadurch gekennzeichnet, daß n = 2 und (x + y) = 1 - 20 ist.

4. Verfahren zur Herstellung von Bernsteinsäure-Derivaten der Formel I gemäß Anspruch 1, dadurch gekennzeichnet, daß man ein Bernsteinsäureanhydrid der allgemeinen Formel II 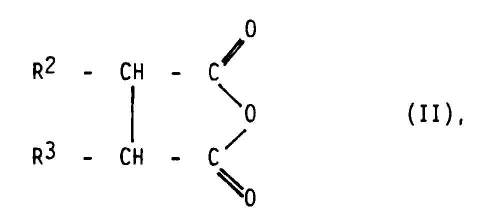 in der eine der Gruppen R² und R³ Wasserstoff und die andere eine Alkenylgruppe mit 12 - 22 C-Atomen ist, mit Alkoholen der Formel R¹ - OH zum Monoester umsetzt, an den Monoester (x + y) mol Ethylenoxid oder Propylenoxid anlagert und gegebenenfalls das Bernsteinsäureanhydrid, den Monoester oder das Oxalkylat zur Absättigung der enthaltenen Doppelbindungen hydriert, wobei R¹, R² und R³, x und y die in Formel I gemäß Anspruch 1 angegebene Bedeutung haben.

5. Verwendung der Bernsteinsäure-Derivate gemäß Formel I nach Anspruch 1 als hautfeuchtigkeitsregulierende Komponente zur Herstellung von kosmetischen oder therapeutischen Zubereitungen zur Behandlung der Haut.

6. Kosmetische und therapeutische Zubereitungen zur Behandlung der Haut, dadurch gekennzeichnet, daß sie als hautfeuchtigkeitsregulierende Komponente ein Bernsteinsäure-Derivat gemäß Formel I nach Anspruch 1 in einer Menge von 1 - 10 Gew.-% in einem geeigneten Träger enthalten.

## Revendications

1. Dérivés de l'acide succinique de formule I,
R¹O(CₙH₂ₙ0)ₓCO - CHR² - CHR³ - CO(OCₙH₂ₙ)_{y}OR⁴ (I),
dans laquelle R représente un radical alcoyle, alcényle, mono- ou dihydroxyalcoyle ou un radical hydroxyalcényle ayant de 6 à 22 atomes de carbone, un des groupes R² et R³, représente de l'hydrogène et l'autre un radical alcoyle ou alcényle ayant de 12 à 22 atomes de carbone,
n = 2 ou 3
x et y des degrés moyens d'oxoalcoylation et
x va de 0 à 20,
y va de 1 à 20,
R⁴ représente de l'hydrogène ou un groupe R¹O-(CₙH₂ₙO)ₓ - CO - CHR² - CHR³ - CO - ou R⁴ a la même signification que R¹.

2. Dérivés de l'acide succinique selon la revendication 1,
caractérisés en ce que
R est un radical alcoyle ayant de 16 à 22 atomes de carbone.

3. Dérivés de l'acide succinique selon la revendication 1 ou la revendication 2,
caractérisés en ce que
n est = 2 et,
(x + y) = 1 à 20.

4. Procédé d'obtention de dérivés de l'acide succinique de formule I selon la revendication 1,
caractérisé en ce qu'
on convertit par réaction un anhydride d'acide succinique de formule générale II, 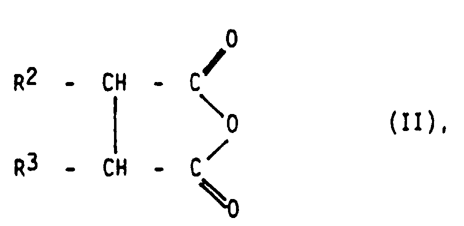 dans laquelle un des groupes R² et R³ est de l'hydrogène et l'autre est un radical alcényle ayant de 12 à 22 atomes de carbone,
avec des alcools de formule R¹-OH, en monoester, on additionne sur le monoester, (x + y) mol d'oxyde d'éthylène ou d'oxyde de propylène et le cas échéant on hydrogène le monoester ou l'oxoalcoylate ou l'anhydride d'acide succinique en vue de la saturation des doubles liaisons qui y sont contenues, dans laquelle R¹, R² et R³, x et y ont les significations indiquées selon la revendication 1.

5. Utilisation des dérivés de l'acide succinique selon la formule I, selon la revendication 1 en tant que composants qui régulent l'humidité de la peau, en vue de l'obtention de préparations cosmétiques et thérapeutiques pour le traitement de la peau.

6. Préparations cosmétiques et thérapeutiques pour le traitement de la peau,
caractérisées en ce qu'
elles renferment comme composants qui régulent l'humidité de la peau, un dérivé de l'acide succinique conformément à la formule I selon la revendication 1, en une quantité allant de 1 à 10 % en poids, dans un support approprié.

## Claims

1. Succinic acid derivatives corresponding to formula I:
R¹O(CₙH₂ₙO)ₓCO - CHR² - CHR³ - CO(OCₙH₂ₙO)_{y}OR⁴ (I)
in which R¹ is an alkyl, alkenyl, mono- or dihydroxyalkyl or hydroxyalkenyl group containing 6 to 22 carbon atoms, one of the substituents R² and R³ is hydrogen and the other is an alkyl or alkenyl group containing 12 to 22 carbon atoms, n = 2 or 3, x and y are average degrees of alkoxylation and have values of 0 to 20 and 1 to 20, respectively, and R⁴ is hydrogen or a group R¹O-(CₙH₂ₙO)ₓ-CO-CHR²-CHR³-CO- or has the same meaning as R¹.

2. Succinic acid derivatives as claimed in claim 1, characterized in that R¹ is an alkyl group containing 16 to 22 carbon atoms.

3. Succinic acid derivatives as claimed in claim 1 or 2, characterized in that n = 2 and (x + y) = 1 to 20.

4. A process for the production of the succinic acid derivatives corresponding to formula I claimed in claim 1, characterized in that a succinic anhydride corresponding to general formula II: 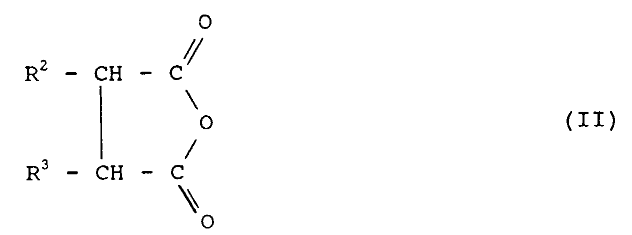 in which one of the substituents R² and R³ is hydrogen and the other is an alkenyl group containing 12 to 22 carbon atoms,
is reacted with alcohols having the formula R¹-OH to form the monoester, (x + y) moles of ethylene oxide or propylene oxide are added onto the monoester and the succinic anhydride, the monoester or the alkoxylate is optionally hydrogenated to saturate the double bonds present, R¹, R² and R³, n, x and y having the meanings defined for formula I in claim 1.

5. The use of the succinic acid derivatives of formula I claimed in claim 1 as a skin-moisture-regulating component for the production of cosmetic or therapeutic skin treatment preparations.

6. Cosmetic and therapeutic skin treatment preparations, characterized in that they contain a succinic acid derivative corresponding to formula I in claim 1 as skin-moisture-regulating component in a quantity of 1 to 10% by weight in a suitable carrier.
